# EUROPÄISCHE PATENTSCHRIFT

(11) **EP 3 352 705 B1**
(45) Veröffentlichungstag und Bekanntmachung des Hinweises auf die Patenterteilung: **22.04.2020**
(21) Anmeldenummer: 16781681.8
(22) Anmeldetag: 20.09.2016
(51) Int. Cl.: A61F 2/07, A61F 2/915

(54) **STENT-GRAFT**
STENT GRAFT
ENDOPROTHÈSE COUVERTE

(30) Priorität: 21.09.2015 DE 102015115891
(43) Veröffentlichungstag der Anmeldung: 01.08.2018
(73) Patentinhaber: Bentley InnoMed GmbH, 72379 Hechingen (DE)
(72) Erfinder: OBRADOVIC, Milisav, 79539 Lörrach (DE); BREGULLA, Rainer, 72336 Balingen (DE)
(74) Vertreter: Hoefer & Partner Patentanwälte mbB
(86) Internationale Anmeldenummer: PCT/EP2016/072212
(87) Internationale Veröffentlichungsnummer: WO 2017/050710

(56) Entgegenhaltungen:
- WO-A2-2012/084202
- DE-A1-102016 100 774

## Beschreibung

Die Erfindung betrifft einen Stent-Graft aus einem Stent mit einer Mehrzahl von nebeneinander angeordneten und durch Verbindungstege miteinander verbundenen Ringsegmenten und einer Membran, wobei die Membran die gesamte Außenseite des Stents abdeckt, um die Stentenden nach innen umgeschlagen ist und zwischen Schlingen von Ringsegmenten und Verbindungsstegen zwischen aneinander grenzenden Ringsegmenten fixiert ist. Ferner betrifft die Erfindung die Verwendung eines solchen Stent-Grafts zur Behandlung vaskulärer Fehlbildungen und ein Verfahren zur Herstellung solcher Stent-Grafts.

Stent-Grafts dieser Art werden in Blutgefäßen eingesetzt, etwa um anomal verengte, erweiterte oder auch geschädigte Blutgefäße zu stützen. Die Kombination von Stent und Membran dient dabei der Versorgung auch ausgedehnterer, behandlungsbedürftiger Gefäßabschnitte, welche eine größere Länge und vor allem auch Flexibilität des Implantats voraussetzen. Insbesondere werden Stent-Grafts bei der Überbrückung von Gefäßfehlbildungen eingesetzt, etwa um Aneurysmen von der Blutzirkulation auszuschließen. Zur Implantation solcher Stent-Grafts werden in der Regel Ballonkatheter verwandt.

Im Stand der Technik ist es bekannt, für diese Aufgabe Stent-Grafts einzusetzen, welche aus zwei Stents und einer flexiblen Membran, beispielsweise aus Teflon bestehen. Einen solchen Stent-Graft beschreibt die Druckschrift EP 2 151 217 A1. Der offenbarte Stent-Graft besteht aus einem inneren Stent und einem koaxial um diesen ersten angeordneten äußeren Stent, zwischen welchen eine flexible, dehnbare Membran angeordnet ist. Die Endbereiche der Stents mit der dazwischen angeordneten Membran werden verschweißt.

Die EP 1 266 635 A2 offenbart einen Stent-Graft, welcher einen zylindrischen Stent und eine zylindrische Membran aufweist, die beispielsweise über Nähte oder Haken miteinander verbunden sind. Zusätzlich oder alternativ kann die Verbindung durch ein geringes Überlappen von Stent und Membran gesichert werden.

Die WO 2009/035679 A1 offenbart einen Stent-Graft, welcher einen durchgehenden inneren Liner aus Polyester oder ePTFE aufweist. An einem Bereich dieses inneren Liners ist ein Stent angeordnet, welcher den inneren Liner koaxial umschließt. Die Bereiche des inneren Liners neben dem Stent sind mit einer zweiten Schicht aus Polyester oder ePTFE ummantelt, um die Wandstärke des Implantats in dem nicht durch den Stent gestützten Bereich zu erhöhen. Die Endbereiche von Stent und zweiter Schicht werden aneinander geschoben. Wenn erforderlich kann zusätzlich Verstärkungsmaterial, z. B. ePTFE, auf die Außenseite der Stent-Graft-Komponenten, insbesondere an den Übergängen zwischen Stent und zweiter Schicht, aufgebracht werden.

Von den genannten Stent-Grafts mit einer Verbindung von Membran und einem Stent verbindet lediglich der in der EP 1 266 635 A2 gezeigte genau einen Stent mit genau einer Membran. Die anderen Lösungen im Stand der Technik erfordern stets einen weiteren Stent oder eine weitere Membran, welche zur Verstärkung koaxial um den ersten Stent bzw. die erste Membran angeordnet sind.

Die Lösung gemäß der EP 1 266 635 A2 verwendet für die Verbindung zwischen Stent und Membran Haken oder Fäden und/oder alternativ ein Überlappen beider Bestandteile. Problematisch ist dabei stets die Haltbarkeit der Haken und Fäden, welche großen Reibungskräften innerhalb des Blutgefäßes ausgesetzt sind. Ebenso besteht die Gefahr der Gewebereizung oder -verletzung durch abstehende Haken oder überstehende Kanten zwischen Stent und Membran.

Stent-Grafts sind ferner in der WO 2012/084202 A1 und der WO 01/66035 A2 beschrieben. Hier wird die Membran an den randständigen Ringsegmente festgelegt, was zur Folge hat, dass die Expansion des Stents bei der Platzierung die Fixierung der Membran am Stent beeinträchtigen kann.

Es ist Aufgabe der vorliegenden Erfindung, einen Stent-Graft zu schaffen, welcher Stent und Membran einfach und haltbar miteinander verbindet, wenig Gefäßlumen verbraucht und keine Gewebereizung oder -verletzung verursacht. Die Verbindung zwischen Membran und Stent soll insbesondere nicht an den Ringsegmenten erfolgen, um eine sichere Fixierung der Membran zu ermöglichen.

Diese Aufgabe löst die Erfindung mit dem Stent-Graft der eingangs bezeichneten Art, bei dem die Membran zwischen wenigstens zwei Schlingen der zweiten Ringsegmente, von den Enden des Stents her gesehen, und den Verbindungsstegen zu den benachbarten endständigen Ringsegmenten fixiert ist.

Die Schlingen oder einzelne Schlingen der zweiten Ringsemente können dabei in Richtung auf das benachbarte randständige Ringsegment verlängert werden.

Das erfindungsgemäße Stent-Graft besteht aus einer Mehrzahl von durch Verbindungsstege miteinander verbundenen mäandrierenden Ringsegmenten. Diese Verbindungsstege und Ringsegmente entsprechen den Ringsegmenten herkömmlicher Stents, wie sie vielfach vorgeschlagen und in Gebrauch sind. Erfindungsgemäß ausgestaltet und verändert sein können lediglich die Ringsegmente, die den endständigen Ringsegmenten benachbart sind, d. h. die zweiten Ringsegmenten von den Stentenden her gesehen.

Unter randständigen Ringsegmenten werden solche Ringsegmente verstanden, die am Ende des Stents angeordnet sind, also den Stent an seinen Enden begrenzen. Wie auch die anderen Ringsegmente haben sie einen mäandrierenden Stegverlauf und sind über Verbindungsstege mit den benachbarten Ringsegmenten (den zweiten Ringsegment) verbunden.

Unter mäandrierendem Stegverlauf der Ringsegmente wird sowohl ein wellenförmiger Stegverlauf als auch ein zickzackförmiger Stegverlauf verstanden. Wellenförmige und zickzackförmige Stegverläufe und Ringsegmente sind bei vaskulären Stents weitgehend üblich, um die bei der Expansion auftretende Ausdehnung aufzufangen.

Zwischen den Ringsegmenten verlaufen übliche Verbindungsstege. Diese Verbindungsstege können einen geraden Verlauf haben und verbinden in diesem Fall entweder die Täler (Innenbögen) benachbarter Ringsegmente miteinander oder alternativ den Bogen einer Schlinge eines Ringsegments mit dem Tal einer Schlinge des benachbarten Ringsegments. Verbindungsstege können aber auch einen gekrümmten Verlauf haben, beispielsweise S-förmig oder spiralförmig aufgewickelt. Die Anordnung und Form dient dazu, die bei der Expansion des Stents auftretende Längenkontraktion zumindest teilweise auszugleichen.

Bögen und Täler der Ringsegmente sind die sich durch den mäandrierenden Verlauf der Ringsegmente ergebenden Spitzen oder Umkehrpunkte der Schlingen der Ringsegmente einerseits und der Eintiefungen andererseits. Jeder Bogen hat zwei benachbarte Täler, jedes Tal zwei benachbarte Bögen. Ein Tal ist ein Innenbogen, die Spitze ein Aussenbogen.

Der erfindungsgemäße Stent-Graft besteht aus einem Stent, der eine Mehrzahl von nebeneinander angeordneten und durch Verbindungsstege miteinander verbundenen Ringsegmenten aufweist, die einen mäandrierenden Verlauf haben. Der Stent ist auf seiner gesamten Außenseite durch eine Membran abgedeckt, die an den Stentenden nach innen umgeschlagen ist und auf der Innenseite - im Lumen - des Stents zwischen Schlingen von Ringsegmenten und venachbarten Verbindungsstegen fixiert ist, dergestalt, dass sich Verbindungsstege auf der einen Seite der Membran und Schlingen der Ringsegmente auf der anderen Seite der Membran befinden.

Vorzugsweise wird die Membran hinter jeder oder jeder zweiten Schlinge des zweiten Ringsegments fixiert.

Erfindungsgemäß ist die Membran an beiden Enden des Stents zwischen den Schlingen des zweiten Ringsegments, von den Enden her gesehen, und den Verbindungsstegen zum endständigen Ringsegment fixiert. Das endständige Ringsegment ist damit auf beiden Seiten von der Membran umgeben.

Um die Fixierung auch während und nach der Expansion zu sichern, ist im erfindungsgemäßen Stent-Graft vorzugsweise zumindest jeweils eine der Schlingen der zweiten Ringsegmente in Richtung auf das jeweilige Stentende hin verlängert, insbesondere jede der Schlingen hinter denen die Membran fixiert ist.

Um eine derartige Art und Weise der Fixierung der Membran zu ermöglichen, enden die Verbindungsstege zum endständigen Ringsegment des Stents im zweiten Ringsegment in einem Schlingental. Dies erlaubt es, die Membran zwischen die Verbindungsstege und die Bögen der Ringsegmente zu schieben und dort festzuhalten. Vorzugsweise sind die Verbindungsstege in diesem Bereiche gerade und gehen von dem stenteinwärts weisenden Bögen der endständigen Ringsegmente aus und führen in die Täler der zweiten Ringsegmente. Alternativ können die Verbindungsstege aber auch die Täler der endständigen Ringsegmente mit den Tälern der zweiten Ringsegmente verbinden.

Zur Verbesserung des Halts der Membran im Bereich des zweiten Ringsegments ist wenigstens eine der Schlingen in Richtung auf das Stentende hin verlängert. Insbesondere sind die stentauswärtsweisenden Schlingen des zweiten Ringsegments alternierend verlängert. Dabei können die längeren der Schlingen der zweiten Ringsegmente um 50 bis 150 % gegenüber den kürzeren verlängert sein.

Diese Verlängerung der Schlingen bewirkt, dass bei der Expansion des Stents die langen Schlingen eine relativ geringe, im Verhältnis zu ihrer Länge, Verkürzung erfahren, während die kürzeren Schlingen sich stark verkürzen. Dies bedeutet, dass der Halt der Membran unter den längeren Schlingen im Wesentlichen erhalten bleibt.

Alternativ oder ergänzend können die stentauswärts weisenden Schlingen des zweiten Ringsegments Blindstege aufweisen, die stentauswärts ausgerichtet sind. Diese Blindstege dienen nicht der Verbindung des Ringsegments zum benachbarten Ringsegment, sie enden frei und können damit zum Halt der Membran beitragen. Ein solcher Blindsteg kann die Schlinge, von dessen Bogen er ausgeht, um 50 bis 100 % verlängern. Dabei können die Blindstege in die Täler der endständigen Ringsegmente hineinragen. Die Blindstege stellen eine Form der Schlingenverlängerung dar, hinter der die Membran fixiert werden kann.

Das erfindungsgemäße Stent-Graft wird in der Regel einen mittels eines Ballonkatheters aufweitbaren Stent, hergestellt beispielsweise aus einem medizinisch akzeptablen Stahl, aufweisen. Alternativ sind auch solche Varianten denkbar, bei welchen der Stent selbstexpandierend ausgebildet ist, z. B. durch Verwendung einer Formgedächtnislegierung, wie Nitinol.

Die Membran, in der Regel eine Folie oder ein Schlauch, kann aus allen in der Medizintechnik üblichen und zugelassenen Materialien bestehen. Insbesondere eignen sich aber PTFE und Polyester. Besonders bevorzugt ist eine Membran aus ePTFE. Die Membran kann dabei zusätzlich funktionell beschichtet sein, z. B. mit entzündungshemmenden, proliferationshemmenden oder therapeutischen Stoffen, beispielsweise mit Rapamycin, Paclitaxel oder Heparin. Die Membran ist vorzugsweise schlauchförmig ausgebildet und weist das notwendige Dehnungsvermögen auf, um bei der Stentexpansion mitzugehen.

Die erfindungsgemäßen Stent-Grafts werden in erster Linie zur Behandlung vaskulärer Fehlbildungen eingesetzt. Dabei kann es sich um das Abschließen von abzweigenden Gefäßen handeln, aber auch um das Stilllegen von Aneurysmen oder arteriovenösen Shunts.

Möglich sind ferner Ausführungsformen, bei denen das erfindungsgemäße Stent-Graft zwei Stenteinheiten aufweist, die dazu dienen, eine dazwischenliegende Schlauchmembran in ein Gefäß einzuspannen. In diesem Fall weist der Stent die Klemmverbindung zur Festlegung der Membran nur an einem Ende auf; das andere Ende der Schlauchmembran ist mit dem zweiten Stent verbunden. Ein solches Stent-Graft kann in großflächig geschädigte Gefäße implantiert werden, beispielsweise nach Obliteration der Epithelzellschicht eines Blutgefäßes.

Die Erfindung betrifft ferner ein Verfahren zur Herstellung der erfindungsgemäßen Stent-Grafts mit den Schritten
Bereitstellen eines Stents mit miteinander verbundenen Ringsegmenten mit mäandrierendem Verlauf;
Einwärtsbiegen wenigstens einer Schlinge eines Ringsegments, das zu einem randständigen Ringsegment benachbart ist;
Aufziehen einer Schlauchfolie auf den Stent;
Umschlagen der Schlauchfolie um wenigstens ein randständiges Ringsegment, so dass das Ende der Schlauchfolie in das Lumen des Stents hineinreicht;
Einschieben des Endes der Schlauchfolie unter die einwärts Schlingen des Ringsegments;
Fixieren des Endes der Schlauchfolie am Stent durch Rückbiegen der einwärts gebogenen Schlingen.

Vorzugsweise wird die Schlauchfolie an beiden Stentenden umgeschlagen und fixiert. Die Fixierung erfolgt vorzugsweise unter mehreren Schlingen der den randständigen Ringsegmenten benachbarten Ringsegmente. Insbesondere dient jede zweite oder jede Schlinge des jeweiligen Ringsegments der Fixierung, d.h. jede zweite oder jede Schlinge des jeweiligen Ringsegments wird aufgebogen und nach Unterschieben der Schlauchfolie wieder zurückgebogen.

Der mit der Schlauchfolie gecoverte Stent kann anschließend in üblicher Weise auf einen Ballon gekrimpt und mithilfe eines Ballonkatheters platziert werden.

Es versteht sich, dass die zum Herstellen des Stent-Grafts verwandte Folie hinreichend dehnbar ist, um bei der Platzierung mit dem Stent aufgeweitet zu werden, ohne zu reißen. Dies ist beispielsweise bei der Verwendung von ePTFE als Material für die Folie oder den Schlauch gegeben.

Die Anmeldung wird durch die beiliegenden Abbildungen, die bevorzugte Ausführungsformen darstellen, näher erläutert. Es zeigen:
- Fig. 1:: ein Stentdesign, das für ein erfindungsgemäßes Stent-Graft eingesetzt werden kann;
- Fig. 2:: ein Foto des Stents gemäß FIG. 1 mit einer erfindungsgemäß befestigten Schlauchfolie;
- Fig. 3:: das Foto eines Stentdesigns mit einwärts gebogener Schlinge im zweiten Ringsegment; und
- Fig. 4:: ein Foto des Stents aus FIG. 3 mit fixiertem Schlauch.

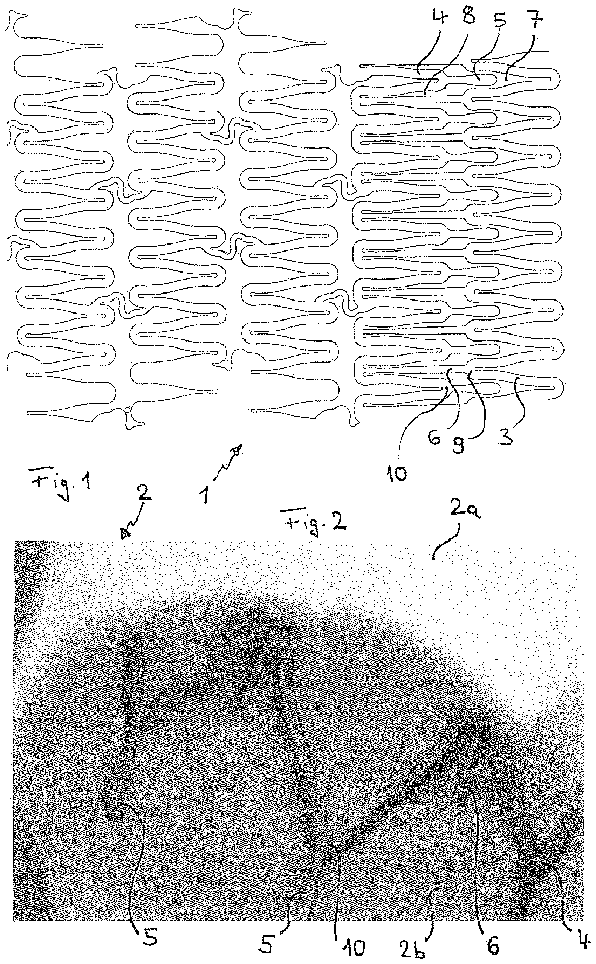

Das Stentdesign von Figur 1 zeigt einen Stent 1 mit mäandrierenden Ringsegmenten 3 und 4 in flächiger Darstellung. Das Ringsegment 3 ist rand-oder endständig, d.h. es bildet den Abschluss des Stents 1. Das benachbarte Ringsegment 4 ist über gerade Stege 6 mit dem randständigen Ringsegment 3 verbunden, wobei die Stege 6 von den Außenbögen 9 der Schlingen des randständigen Ringsegments 3 ausgehend in die Innenbögen 8 des benachbarten Ringsegments 4 reichen.

Die Schlingen des zweiten Ringsegments 4 weisen an ihren Außenbögen 10 Blindstege 5 auf, die keinerlei Verbindung zu dem benachbarten Ringsegment 3 herstellen, vielmehr blind enden. Diese Blindstege 5 dienen der Verlängerung der Schlingen des zweiten Ringsegments 4 - bei der Expansion des Stents 1 strecken sich die Schlingen und reichen in diesem gestreckten Zustand nicht mehr aus, eine dahinter fixierte Abdeckung festzuhalten. Die Blindstege 5 vergrößern den Kontakt zur Abdeckung und verbessern den Halt.

Im dargestellten Fall reichen die Blindstege 5 in die Innenbögen 7 des randständigen Ringsegments 3 hinein. Sie haben in etwa 50 % der Länge der Schlingen des zweiten Ringsegments 4, wenn der Stent nicht expandiert ist.

Figur 2 zeigt ein Foto des Stents 1 von Figur 1 mit daran fixierter Abdeckung 2, wobei die Außenseite der Abdeckung 2 mit 2a bezeichnet ist und das um das Stentende herumgeschlagene Ende der Abdeckung 2 mit 2b. Das Foto ist von der Stirnseite des Stents 1 her in das Lumen hinein aufgenommen worden.

Die Abdeckung 2 ist im dargestellten Fall eine Schlauchfolie aus ePTFE, die über den Stent gezogen wird, mit ihrem Ende 2b um das Stentende herumgeführt wird und zwischen den Außenbögen 10 mit den Blindstegen 5 des zweiten Ringsegments 4 einerseits und den Verbindungsstegen 6 zwischen den Ringsegmenten 3 und 4 andererseits festgeklemmt ist. Die Schlingen des zweiten Ringsegments 4 sind durch die Expansion des Stents 1 deutlich aufgeweitet.

Grundsätzlich ist die Schlauchfolie 2 an beiden Enden des Stents 1 umgeschlagen und auf die oben beschriebene Art und Weise fixiert. Es sind aber Anwendungen denkbar, bei denen eine Fixierung an nur einem Stentende ausreichend ist.

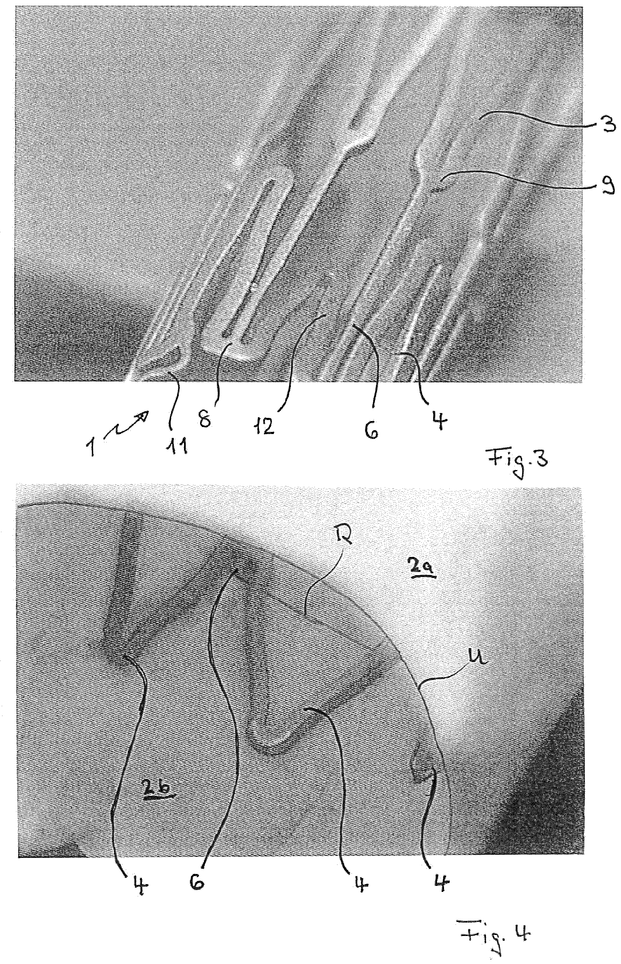

Figur 3 erläutert die Art und Weise der Fixierung der Abdeckung 2 im Lumen des Stents 1 anhand eines Fotos entlang der Stentachse. Das Stentende befindet sich am oberen Rand. Das zweite Ringsegment 4 ist wie in Figur 1 dargestellt über Verbindungsstege 6 mit den Außenbögen neun des ersten Ringsegments 3 verbunden. Die im weiteren Stentverlauf vorhandenen Ringsegmente sind über S-förmig ausgebildete Stege 12 miteinander verknüpft. Eine der Schlingen des Ringsegments 4, hier mit der Bezugsziffer 12 bezeichnet, ist nach innen umgebogen. Dies erleichtert das unterschieben des Endes eine Abdeckung, insbesondere einer Schlauchfolie 2, die anschließend durch Rückbiegen der umgebogenen Schlinge 12 festgelegt wird. In der Praxis wird für die Fixierung mehr als nur eine Schlinge umgebogen; vorzugsweise ist dies bei jeder oder jeder zweiten Schlinge der Fall.

Figur 4 zeigt den Stent von Figur 3 anhand eines Fotos analog zu Fig. 2 mit daran fixierter Schlauchfolie 2, die zwischen inzwischen zurückgebogenen Schlingen des Ringsegments 4 und Verbindungsstegen 6 festgelegt ist. 2 a bezeichnet den außen gelegenen Teil der Schlauchfolie 2, 2 b den umgeschlagenen Teil. Der Verbindungssteg 6 ist im Ansatz zu erkennen. 11 bezeichnet den Umschlagsrand der Schlauchfolie 2/2 a, R den Rand des umgeschlagenen und fixierten Teils 2 b.

Es versteht sich, dass die hier dargestellten Ausführungsformen lediglich Beispiele sind und dass die einzelnen Merkmale dieser beispielhaften Ausführungsformen in jedweder Kombination in den erfindungsgemäßen Stent-Grafts vorkommen können.

## Patentansprüche

1. Stent-Graft aus einem Stent (1) mit einer Mehrzahl von nebeneinander angeordneten und durch Verbindungsstege (6) miteinander verbundenen Ringsegmenten (3, 4) mit mäandrierendem Verlauf und wenigstens einer Membran (2), wobei die Membran die gesamte Außenseite des Stents (1) abdeckt, um die Stentenden nach innen umgeschlagen ist und zwischen Schlingen von Ringsegmenten (4) und Verbindungsstegen (6), die aneinandergrenzende Ringsegmenten (3, 4) miteinander verbinden, fixiert ist, **dadurch gekennzeichnet, dass** die Membran (2) zwischen wenigstens zwei Schlingen des zweiten Ringsegments (4), von den Enden des Stents (1) her gesehen, und den Verbindungsstegen (6) zu dem benachbarten endständigen Ringsegment (3) fixiert ist.

2. Stent-Graft nach Anspruch 1, **dadurch gekennzeichnet, dass** die endständigen Ringsegmente (3) über gerade Verbindungsstege (6) mit den benachbarten Ringsegmenten (4) verbunden sind, wobei die geraden Verbindungstege (6) von den stenteinwärts weisenden Aussenbögen (9) der endständigen Ringsegmente (3) in die Täler oder Innenbögen (8) der zweiten Ringsegmente (4) führen.

3. Stent-Graft nach Anspruch 1 oder 2, **dadurch gekennzeichnet, dass** die stentauswärtsweisenden Schlingen der zweiten Ringsegmente (4) alternierend eine unterschiedliche Länge aufweisen.

4. Stent-Graft nach Anspruch 3, **dadurch gekennzeichnet, dass** das Längenverhältnis der kurzen zu den langen Schlingen der zweiten Ringsegmente 1:2 bis 2:3 beträgt.

5. Stent-Graft nach Anspruch 1 oder 2, **dadurch gekennzeichnet, dass** die auswärtsweisenden Bögen (10) der zweiten Ringsegmente (4) durch Blindstege (5) stentauswärts verlängert sind.

6. Stent-Graft nach Anspruch 5, **dadurch gekennzeichnet, dass** die Blindstege (5) eine Länge aufweisen, die 50 bis 100 % der Länge der die Blindstege (5) tragenden Schlingen (4) ausmacht.

7. Stent-Graft nach Anspruch 5 oder 6, **dadurch gekennzeichnet, dass** die Blindstege (5) in die Täler (7) der endständigen Ringsegmente (3) hineinragen.

8. Stent-Graft nach einem der vorstehenden Ansprüche, **dadurch gekennzeichnet, dass** der Stent (1) eine Membran (2) in Form eines Schlauches aufweist.

9. Stent-Graft nach einem der vorstehenden Ansprüche, **dadurch gekennzeichnet, dass** die Membran (2) aus ePTFE-Schlauch besteht.

10. Stent-Graft nach einem der vorstehenden Ansprüche, **dadurch gekennzeichnet, dass** der Stent (1) ein Ballon-expandierbarer Stent ist.

11. Stent-Graft nach Anspruch 10, aufgekrimpt auf den Ballon eines Ballonkatheters.

12. Stent-Graft nach einem der Ansprüche 1 bis 9 mit einem selbst-expandierenden Stent aus einer Formgedächtnislegierung, insbesondere Nitinol.

13. Verfahren zur Herstellung eines Stent-Grafts nach einem der Ansprüche 1 bis 10 mit den Schritten:
Bereitstellen eines Stents (1) mit miteinander verbundenen Ringsegmenten (3,4) mit mäandrierendem Verlauf, wobei die Ringsegmenten (3,4) eine Mehrzahl von Schlingen aufweisen;
Einwärtsbiegen wenigstens einer Schlinge (12) eines Ringsegments (4), das zu einem randständigen Ringsegment (3) benachbart ist;
Aufziehen einer Schlauchmembran (2) auf den Stent (1);
Umschlagen der Schlauchmembran (2) um wenigstens ein randständiges Ringsegment (3), so dass das Ende der Schlauchmembran (2) in das Lumen des Stents (1) hineinreicht;
Einschieben des Endes (2b) der Schlauchmembran (2) unter die einwärts weisenden Schlingen (12) des Ringsegments (4); und
Fixieren des Endes (2b) der Schlauchmembran (2) am Stent (1) durch Rückbiegen der einwärts gebogenen Schlingen (12).

14. Verfahren nach Anspruch 13, **dadurch gekennzeichnet, dass** die Schlauchmembran (2) an beiden Stentenden umgeschlagen und fixiert wird.

15. Verfahren nach Anspruch 14, **dadurch gekennzeichnet, dass** die Fixierung unter mehreren Schlingen (12) der den randständigen Ringsegmenten (3) benachbarten Ringsegmente (4) erfolgt.

16. Verfahren nach Anspruch 15, **dadurch gekennzeichnet, dass** die Fixierung unter jeder oder jeder zweiten Schlinge(12) der Ringsegmente (3) erfolgt.

17. Verfahren nach einem der Ansprüche 13 bis 16, **dadurch gekennzeichnet, dass** das mit der Schlauchmembran (2) versehenen Stent-Graft auf einen Ballon gekrimpt wird.

## Claims

1. A stent graft comprising a stent (1) with a plurality of ring segments (3, 4) with a meandering course arranged side by side and interconnected by connecting webs (6) and at least one membrane (2), the membrane covering the entire outside of the stent (1), being turned inwards around the stent ends, and being fixed between loops of ring segments (4) and connecting webs (6) interconnecting adjacent ring segments (3, 4),
**characterised in that** the membrane (2) is fixed between at least two loops of the second ring segment (4), as seen from the ends of the stent (1), and the connecting webs (6) to the adjacent terminal ring segment (3).

2. The stent graft according to claim 1, **characterised in that** the terminal ring segments (3) are connected with the adjacent ring segments (4) via straight connecting webs (6), wherein the straight connecting webs (6) lead from the outer arches facing stent-inwardly (9) of the terminal ring segments (3) into the valleys or inner arches (8) of the second ring segments (4).

3. The stent graft according to claim 1 or 2, **characterised in that** the loops facing stent-outwardly of the second ring segments (4) alternatingly have a different length.

4. The stent graft according to claim 3, **characterised in that** the length ratio of the short to the long loops of the second ring segments is 1:2 to 2:3.

5. The stent graft according to claim 1 or 2, **characterised in that** the outwardly facing arches (10) of the second ring segments (4) are elongated stent-outwardly by dummy webs (5).

6. The stent graft according to claim 5, **characterised in that** the dummy webs (5) have a length corresponding to 50 to 100 % of the length of the loops (4) carrying the dummy webs (5).

7. The stent graft according to claim 5 or 6, **characterised in that** the dummy webs (5) protrude into the valleys (7) of the terminal ring segments (3).

8. The stent graft according to any one of the preceding claims, **characterised in that** the stent (1) has a membrane (2) in the form of a tube.

9. The stent graft according to any one of the preceding claims, **characterised in that** the membrane (2) consists of ePTFE tubing.

10. The stent graft according to any one of the preceding claims, **characterised in that** the stent (1) is a balloon-expandable stent.

11. The stent graft according to claim 10, crimped onto the balloon of a balloon catheter.

12. The stent graft according to any one of claims 1 to 9 with a self-expanding stent made of a shape-memory alloy, in particular nitinol.

13. A method for manufacturing a stent graft according to any one of claims 1 to 10, comprising the steps of:
providing a stent (1) having interconnected ring segments (3, 4) with a meandering course, the ring segments (3, 4) having a plurality of loops;
bending inwardly at least one loop (12) of a ring segment (4) adjacent to a marginal ring segment (3);
drawing a tubular membrane (2) onto the stent (1);
turning the tubular membrane (2) around at least one marginal ring segment (3), so that the end of the tubular membrane (2) extends into the lumen of the stent (1);
inserting the end (2b) of the tubular membrane (2) under the inwardly facing loops (12) of the ring segment (4); and
fixing the end (2b) of the tubular membrane (2) to the stent (1) by bending back the inwardly bent loops (12).

14. The method according to claim 13, **characterised in that** the tubular membrane (2) is turned around and fixed at both stent ends.

15. The method according to claim 14, **characterised in that** the fixing is undertaken under a plurality of loops (12) of the ring segments (4) adjacent to the marginal ring segments (3).

16. The method according to claim 15, **characterised in that** the fixing is undertaken under every or every second loop (12) of the ring segments (3).

17. The method according to any one of claims 13 to 16, **characterised in that** the stent graft provided with the tubular membrane (2) is crimped onto a balloon.

## Revendications

1. Endoprothèse couverte composée d'une endoprothèse (1) avec une pluralité de segments annulaires (3, 4) agencés les uns à côté des autres et reliés entre eux par des nervures de liaison (6), avec un tracé en méandre et au moins une membrane (2), dans laquelle la membrane recouvre le côté extérieur entier de l'endoprothèse (1), est repliée autour des extrémités d'endoprothèse vers l'intérieur et est fixée entre des boucles de segments annulaires (4) et des nervures de liaison (6) qui relient des segments annulaires (3, 4) contigus les uns aux autres entre eux, **caractérisée en ce que** la membrane (2) est fixée entre au moins deux boucles du second segment annulaire (4), vu depuis les extrémités de l'endoprothèse (1), et les nervures de liaison (6) avec le segment annulaire (3) terminal contigu.

2. Endoprothèse couverte selon la revendication 1, **caractérisée en ce que** les segments annulaires (3) terminaux sont reliés par le biais de nervures de liaison droites (6) aux segments annulaires (4) contigus, dans laquelle les nervures de liaison (6) droites mènent des arcs extérieurs (9) dirigés vers l'intérieur de l'endoprothèse des segments annulaires (3) terminaux dans les creux ou arcs intérieurs (8) des seconds segments annulaires (4).

3. Endoprothèse couverte selon la revendication 1 ou 2, **caractérisée en ce que** les boucles dirigées vers l'extérieur de l'endoprothèse des seconds segments annulaires (4) présentent en alternance une longueur différente.

4. Endoprothèse couverte selon la revendication 3, **caractérisée en ce que** le rapport de longueur des boucles courtes à longues des seconds segments annulaires est de 1:2 à 2:3.

5. Endoprothèse couverte selon la revendication 1 ou 2, **caractérisée en ce que** les arcs dirigés vers l'extérieur (10) des seconds segments annulaires (4) sont rallongés vers l'extérieur de l'endoprothèse par des nervures borgnes (5).

6. Endoprothèse couverte selon la revendication 5, **caractérisée en ce que** les nervures borgnes (5) présentent une longueur qui représente 50 à 100 % de la longueur des boucles (4) portant les nervures borgnes (5).

7. Endoprothèse couverte selon la revendication 5 ou 6, **caractérisée en ce que** les nervures borgnes (5) pénètrent dans les creux (7) des segments annulaires (3) terminaux.

8. Endoprothèse couverte selon l'une des revendications précédentes, **caractérisée en ce que** l'endoprothèse (1) présente une membrane (2) en forme de tuyau.

9. Endoprothèse couverte selon l'une des revendications précédentes, **caractérisée en ce que** la membrane (2) se compose d'un tuyau en ePTFE.

10. Endoprothèse couverte selon l'une des revendications précédentes, **caractérisée en ce que** l'endoprothèse (1) est une endoprothèse à ballonnet expansible.

11. Endoprothèse couverte selon la revendication 10, sertie sur le ballonnet d'un cathéter à ballonnet.

12. Endoprothèse couverte selon l'une des revendications 1 à 9, avec une endoprothèse autoexpansible en un alliage à mémoire de forme, en particulier en nitinol.

13. Procédé de fabrication d'une endoprothèse couverte selon l'une des revendications 1 à 10 avec les étapes :
la mise à disposition d'une endoprothèse (1) avec des segments annulaires (3, 4) reliés entre eux avec un tracé en méandre, dans lequel les segments annulaires (3, 4) présentent une pluralité de boucles ;
le pliage vers l'intérieur au moins d'une boucle (12) d'un segment annulaire (4) qui est contigu à un segment annulaire (3) marginal ;
l'enfilage d'une membrane de tuyau (2) sur l'endoprothèse (1) ;
le repli de la membrane de tuyau (2) autour au moins d'un segment annulaire (3) marginal de sorte que l'extrémité de la membrane de tuyau (2) atteigne le lumen de l'endoprothèse (1) ;
l'enfoncement de l'extrémité (2b) de la membrane de tuyau (2) sous les boucles (12) dirigées vers l'intérieur du segment annulaire (4) ; et
la fixation de l'extrémité (2b) de la membrane de tuyau (2) à l'endoprothèse (1) par rétroflexion des boucles (12) pliées vers l'extérieur.

14. Procédé selon la revendication 13, **caractérisé en ce que** la membrane de tuyau (2) est repliée et fixée aux deux extrémités de l'endoprothèse.

15. Procédé selon la revendication 14, **caractérisé en ce que** la fixation est effectuée sous plusieurs boucles (12) des segments annulaires (4) contigus aux segments annulaires (3) marginaux.

16. Procédé selon la revendication 15, **caractérisé en ce que** la fixation est effectuée sous chaque ou chaque deuxième boucle (12) des segments annulaires (3).

17. Procédé selon l'une des revendications 13 à 16, **caractérisé en ce que** l'endoprothèse couverte pourvue de la membrane de tuyau (2) est sertie sur un ballonnet.
